# EUROPEAN PATENT SPECIFICATION

(11) **EP 2 840 397 B1**
(45) Date of publication and mention of the grant of the patent: **29.06.2016**
(21) Application number: 14182505.9
(22) Date of filing: 25.11.2011
(51) Int. Cl.: G01N 33/68

(54) **Method of diagnosing down's syndrome**
Verfahren zur Diagnose von Down-Syndrom
Méthode pour diagnostiquer le syndrome de down

(30) Priority: 26.11.2010 GB 201020071
(43) Date of publication of application: 25.02.2015
(62) Divisional of application: 11808272.6
(73) Proprietor: University of Plymouth Enterprise Limited, Plymouth Devon PL4 8AA (GB)
(72) Inventor: Madgett, Tracey Elizabeth, Plymouth, Devon PL6 6AZ (GB); Avent, Neil David, Plymouth, Devon PL8 2EL (GB)
(74) Representative: Henderson, Helen Lee

(56) References cited:
- US-A1- 2008 176 239
- B. WEISZ: "An update on antenatal screening for Down's syndrome and specific implications for assisted reproduction pregnancies", HUMAN REPRODUCTION UPDATE, vol. 12, no. 5, 1 January 2006 (2006-01-01), pages 513-518, XP055018739, ISSN: 1355-4786, DOI: 10.1093/humupd/dml021
- AVENT ET AL: "Post-genomics studies and their application to non-invasive prenatal diagnosis", SEMINARS IN FETAL AND NEONATAL MEDICINE, ELSEVIER, GB, vol. 13, no. 2, 4 February 2008 (2008-02-04), pages 91-98, XP022472012, ISSN: 1744-165X, DOI: 10.1016/J.SINY.2007.12.011

## Description

### Field of the invention

This invention relates to the diagnosis of Down's Syndrome, in particular, diagnostic markers for the non-invasive prenatal diagnosis of Down's Syndrome.

### Background

Down's syndrome is a relatively common chromosomal abnormality that causes mental retardation. It results from an extra copy (of which there are normally 2) of chromosome 21. The condition is referred to as trisomy 21 because of this. All pregnancies are assessed for their potential risk for chromosomal abnormality (aneuploidy) by a series of screening tests for maternal plasma proteins that are fetally-derived via the placenta. Down's syndrome increases in incidence with increasing maternal age, and is presently screened for by certain maternal serum biomarkers including alpha fetoprotein (AFP), |3-HCG and PAPP-A.

Unfortunately these tests are not fully diagnostic, each individual test is at best able to predict 85% accurately if the fetus being carried has Down's syndrome, there is also a 5-10% false positive prediction rate. A high-risk score for any of these markers may then be followed up by assessment of nuchal translucency, and then by invasive prenatal diagnosis to sample fetal cells to enable either karyotyping or quantitative fluorescent PCR to assess chromosomal numbers.

These invasive procedures impart a small but significant risk of procedural related loss due to spontaneous miscarriage. Significant efforts have been expended in seeking non-invasive alternatives to Down syndrome diagnostics. In recent years, much emphasis has been placed on using free fetal DNA and more recently free fetal mRNA found in maternal plasma. Despite these advances, a protein-based assay with greater screening and perhaps even diagnostic potential would make cheap and technically non-challenging alternative to the use of fetal derived nucleic acids.

There is, therefore, a need to provide diagnostic markers for Down's Syndrome which can be detected non-invasively.

Weisz and Rodeck (Human Reproduction Update, Vol. 12, No. 5, pp 513-518, 2006) discloses diagnostic serum protein markers for Down's syndrome in pregnant women.

### Summary of the Invention

The invention is the method defined by claim 1. Other markers and methods are provided as disclosure but are not claimed.

### Summary of the Disclosure

According to a first aspect of the disclosure there is provided a method of diagnosing Down's Syndrome the method comprising identifying a different expression pattern of at least one diagnostic marker in the blood, plasma or serum of a patient compared to the normal expression pattern of the marker, characterised in that the diagnostic marker is selected from those shown in Table 1.

**Table 1**

| Marker Number | Marker name |
|---|---|
| 1 | taste receptor type 1 member 1 |
| 2 | Zinc finger protein 704 |
| 3 | ATP synthase 0 subunit, mitochondrial precursor |
| 4 | 1-acylglycerol-3 -phosphate O-acyltransferase 3, isoform CRAb |
| 5 | Tripartite motif protein 46 |
| 6 | Myelin protein zero-like protein 2 precursor |
| 7 | ATP synthase coupling factor 6, mitochondrial precursor |
| 8 | 80 kDa MCM3-associated protein |
| 9 | Submaxillary gland androgen-regulated protein 3 homolog A precursor |
| 10 | Human cDNA clone |
| 11 | AP2-associated protein kinase 1 |
| 12 | Orphan nuclear receptor NR1D1 |
| 13 | Cystatin-B (Stefin-B) (Liver thiol proteinase inhibitor) |
| 14 | glial cell derived neurotrophic factor |
| 15 | Heat-shock protein beta-9 (HspB9). |
| 16 | MORC family CW-type zinc finger protein 3 |
| 14 | Serine protease inhibitor Kazal-type 7 precursor |
| 18 | Receptor-interacting serine/threonine-protein kinase 4 |
| 19 | Suppressor of cytokine signaling 1 |
| 20 | cDNA clone DKFZp434C2331 |
| 21 | Dual specificity protein phosphatase 15 |
| 22 | Trinucleotide repeat-containing protein 18 |
| 23 | Ubiquitin carboxyl-terminal hydrolase 16 |
| 24 | Paired mesoderm homeobox protein 2A |
| 25 | Serine/threonine-protein kinase 11 |
| 26 | Ribosome-binding protein 1 |
| 27 | Transcribed locus BX090181 |
| 28 | ATP synthase coupling factor 6, mitochondrial precursor |
| 29 | Zinc finger protein 488 |
| 30 | Uncharacterized protein C16orf3 |
| 31 | Pericentrin (Pericentrin B) (Kendrin). |
| 32 | E4F transcription factor 1 |
| 33 | v-ets erythroblastosis virus E26 oncogene homolog 2 (avian) |
| 34 | spire homolog 2 |
| 35 | Periodic tryptophan protein 2 homolog |
| 36 | Glycinamide ribonucleotide synthetase |
| 37 | HemK methyltransferase family member 2 |
| 38 | Trypsin-like serine protease |
| 39 | RRPl-like protein (Protein NNP-1) |
| 40 | Immunoglobulin heavy chain V gene segment |
| 41 | Coiled-coil-helix-coiled-coil-helix domain-containing protein 2 |
| 42 | DNA polymerase kappa |
| 43 | Inositol hexakisphosphate kinase 2 |
| 44 | Zinc finger protein 625 |
| 45 | leptin receptor |
| 46 | Blood vessel epicardial substance |
| 47 | Uncharacterized protein C13orf30 |
| 48 | Inhibitor of growth protein 5 |
| 49 | Zinc finger CCCH domain-containing protein 5. |
| 50 | Rhodopsin (Opsin-2). |
| 51 | Meiotic recombination protein DMC1/LIM15 homolog |
| 52 | CD lb molecule |
| 53 | Putative uncharacterized protein DKFZp761E198 |
| 54 | Ciliary dynein heavy chain 8 |

The term "different expression pattern", as used throughout this specification, indicates that the expression of a diagnostic marker in the blood or serum of the test subject is different to the normal expression level of that marker ie different to the levels found in the blood, plasma or serum of pregnant women whose fetuses do not have Down's Syndrome.

The term "an increased amount", as used throughout this specification, indicates that the amount of diagnostic marker in the blood or serum of a pregnant woman, is greater than normal i.e. greater than the levels found in the blood, plasma or serum of pregnant women whose fetuses do not have Down's Syndrome.

The term "a decreased amount", as used throughout this specification, indicates that the amount of a diagnostic in the blood or serum of a pregnant woman is less than the normal i.e. less than the levels found in the blood, plasma or serum of pregnant women whose fetuses do not have Down's Syndrome.

According to the second aspect of the disclosure there is provided a method of diagnosing Down's Syndrome, the method comprising identifying an increased amount of at least one diagnostic marker in the blood, plasma or serum of a patient compared to the normal expression pattern of the marker, characterised in that the diagnostic marker is selected from markers number 1 to 39 inclusive of those shown in Table 1.

According to the third aspect of the disclosure there is provided a method of diagnosing Down's Syndrome, the method comprising identifying a decreased amount of at least one diagnostic marker in the blood, plasma or serum of a patient compared to the normal expression pattern of the marker, characterised in that the diagnostic marker is selected from markers number 40 to 54 inclusive of those shown in Table 1.

### Methods

Analysis the expression of genes in placental tissue obtained with full ethical consent from women who chose to terminate both normal and Down's syndrome fetuses.

Gene expression of placental tissue obtained with full ethical consent from women who chose to terminate both normal and Down syndrome fetuses was undertaken. The samples were classified according to the parameters karyotype, gestational age and gender. Among these, the karyotype may adopt the values *T21, Norma*/-*N* or *N,* while the samples were either derived from female (F) or male (M) fetal chorionic villi. All samples are from time points near the first-to-second trimester transition and are detailed in Table 2.

**Table 2. Sample details**

| Sample Number | Karyotype | Sex | Gestational age (week.days) |
|---|---|---|---|
| 1 | Normal | M | 12.6 |
| 2 | Normal | M | 12.1 |
| 3 | Normal | M | 12.4 |
| 4 | Normal | M | 12.2 |
| 5 | Normal | M | 13.4 |
| 6 | Normal | M | 12.2 |
| 7 | Normal | M | 13.1 |
| 8 | Normal | M | 12.4 |
| 9 | Normal | M | 12.6 |
| 10 | Normal | F | 13.2 |
| 11 | Normal-N | M | 12.2 |
| 12 | Normal-N | M | 12.3 |
| 13 | Normal-N | M | 13.3 |
| 14 | Normal-N | M | 12.6 |
| 15 | Normal-N | M | 14 |
| 16 | Normal-N | F | 13 |
| 17 | Normal-N | M | 12.6 |
| 18 | Normal-N | M | 12.3 |
| 19 | Normal-N | M | 12.4 |
| 20 | Normal-N | M | 12.6 |
| 21 | T21 | M | 12.5 |
| 22 | T21 | M | 12.4 |
| 23 | T21 | F | 12.2 |
| 24 | T21 | F | 12.6 |
| 25 | T21 | F | 13 |
| 26 | T21 | F | 12.2 |
| 27 | T21 | M | 12.3 |
| 28 | T21 | F | 13.4 |
| 29 | T21 | M | 13.3 |
| 30 | T21 | F | 12.6 |
| 31 | T21 | M | 12.4 |
| 32 | T21 | F | 12.1 |
| 33 | T21 | M | 13.5 |
| 34 | T21 | M | 13.6 |
| 35 | T21 | M | 13 |
| 36 | T21 | F | 12.2 |
| 37 | T21 | M | 12.1 |
| 38 | T21 | F | 12.2 |
| 39 | T21 | F | 13.1 |
| 40 | T21 | F | 12.6 |

40 microarray datasets were generated by single-color hybridization of human RNAs on Agilent Whole Human Genome Oligo Microarrays after T7 RNA amplification. The samples were derived from chorionic villous samples at a gestational age between week 12 and 14. Each sample represents a different donor and fetuses were either male or female. The 40 microarray datasets were subdivided into three karyotype classes (*T21*, *Normal* and *Norma*/-*N*). While *Normal* denotes fetuses with normal karyotype but a conspicuous maternal serum marker indicative of trisomy 21, *Norma*/-*Nhas* both a normal karyotype and normal maternal serum markers. Hence, *Norma*/*-N* labeled samples were used as control.

Ratios were computed using the RosettaResolver™ Software (Rosetta Inpharmatics). A common reference (CR) was computed by creating an artificial pool of all *Normal-N* samples. All ratio data were transformed to logarithms to the base 2 (log2 ratio). In addition, for each ratio the corresponding 'fold-change' was computed for a more intuitive understanding of the expression changes.

The unfiltered expression ratios (based on the common reference) of all 40 samples in this analysis were compared in a correlation analysis.

Discriminatory gene analysis (DGA) was undertaken to test each gene for expression differences between the comparison groups. A two-group t-test was performed comparing the groups pairwise, requiring a Bonferroni-corrected p-value of 0.05 or better.

Ratios were computed using the RosettaResolver™ Software (Rosetta Inpharmatics). A common reference (CR) was computed by creating an artificial pool of all *Normal-N* samples.

### Results

We selected the most highly up-regulated or down- regulated genes. These are shown in Tables 3 and 4 below many of these are soluble proteins which will be found in maternal blood and thus available as diagnostic markers.

**Table 3 Genes highly upregulated in Down's Syndrome samples (T21)**

| Marker **No** | **SeqName** | **Gene description / name** | **Seqcode** | **Accession No.** | **Position** |
|---|---|---|---|---|---|
| 1 | TAS1R1 | taste receptor type 1 member 1 | A 23 PI60886 | AL591866 | Chr1 |
| 2 | ZNF704 | Zinc finger protein 704 | A 24 P230074 | AK131274 | Chr8 |
| 3 | ATP50 | ATP synthase 0 subunit, | A 23 P143474 | AK222608 | Chr21 |
| 4 | AGPAT3 | 1-acylglycerol-3-phosphate O-acvltransferase 3, | A 23 P356466 | AK074300 | Chr21 |
| 5 | TRIM46 | Tripartite motif protein | A 23 P46222 | AK026882 | Chr1 |
| 6 | MPZL2 | Myelin protein zero-like protein 2 | A 23 PI50379 | BC017774 | Chr 11 |
| 7 | ATP5J | ATP synthase coupling factor 6, mitochondrial | A 24 P745670 | AL110183 | Chr21 |
| 8 | MCM3AP | 80 kDa MCM3- | A 23 P120744 | AB005543 | Chr 21 |
| | | protein | | | |
| 9 | SMR3A | Submaxillary gland androgen-regulated protein 3 homolog A | A 23 P41365 | AC 106884 | Chr4 |
| 10 | BC009749 | Human cDNA clone | A 24 P592318 | BC009749 | Chr9 |
| 11 | AAK1 | AP2-associated protein kinase 1 | A 23 P209826 | AB028971 | Chr2 |
| 12 | NR1D1 | Orphan nuclear receptor NR1D1 | A 24 P250227 | BC047875 | Chr17 |
| 13 | CSTB | Cystatin-B (Stefin-B) (Liver thiol proteinase | A 23 PI54894 | AB083085 | Chr21 |
| 14 | GDNF-002 | glial cell derived neurotrophic | A 24 P376451 | AF053748 | Chr5 |
| 15 | HSPB9 | Heat-shock protein beta-9 (HspB9). | A 23 P416212 | AJ302068 | Chr17 |
| 16 | MORC3 | MORC family CW-type zinc finger | A 23 P325501 | AP000692 | Chr21 |
| 14 | SPINK7 | Serine protease inhibitor Kaza1-type 7 | A 23 P213832 | AF268198 | Chr5 |
| 18 | RIPK4 | Receptor-interacting serine/threonine-protein kinase 4 | A 24 P125871 | AB047783 | Chr21 |
| 19 | SOCS1 | Suppressor of cytokine signaling 1 | A 24 P48014 | AB000676 | Chr16 |
| 20 | AL137495 | cDNA clone DKFZp434C23 | A 24 P655646 | AL137495 | Chr4 |
| 21 | DUSP15 | Dual specificity protein phosphatase | A 23 P154771 | AL160175 | Chr20 |
| 22 | TNRC18 | Trinucleotide repeat-containing | A 24 P75245 | U80753 | Chr7 |
| 23 | USP16 | Ubiquitin carboxyl-terminal hydrolase 16 | A 23 P257911 | AF113219 | Chr21 |
| 24 | PHOX2A | Paired mesoderm homeobox protein 2A | A 24 P215445 | AF022722 | Chrl1 |
| 25 | STK11 | Serine/threonine-protein kinase 11 | A 23 PI6483 | U63333 | Chrl9 |
| 26 | RRBP1 | Ribosome-binding | A 32 P28309 | AI916036 | Chr20 |
| 27 | BX090181 | Transcribed locus | A 32 P9518 | BX090181 | |
| 28 | ATP5J | ATP synthase coupling factor 6, mitochondrial | A 23 PI54832 | AL110183 | Chr21 |
| 29 | ZNF488 | Zinc finger protein 488 | A 24 P398210 | BC051323 | ChrlO |
| 30 | C16orf3 | Uncharacterized protein C16orf3 | A 23 P344515 | AF050080 | Chrl6 |
| 31 | PCNT | Pericentral (Pericentral B) (Kendrin). | A 23 P57347 | AK024009 | Chr21 |
| 32 | E4F1 | E4F transcription factor | A 24 P205100 | NM 004424.3 | Chrl6 |
| 33 | ETS2 | v-ets erythroblastosis virus E26 oncogene homolog 2 (avian) | A 23 P257924 | NM 005239 | Chr21 |
| 34 | SPIRE2 | spire homolog 2 | A 24 P544996 | AJ422077 | Chrl6 |
| 35 | PWP2 | Periodic tryptophan protein 2 homolog | A 23 PI02925 | AB001517 | Chr21 |
| 36 | GART | Glycinamide ribonucleotide | A 23 P80098 | AB208785 | Chr21 |
| 37 | N6AMT1 | HemK methyltransferase | A 23 P80086 | AF139682 | Chr21 |
| | | family member 2 | | | |
| 38 | LOC646960 | Trypsin-like serine | A 24 P15182 | NT 005403.17 | Chr21 |
| 39 | RRP1 | RRPl-like protein (Protein NNP-1) | A 23 P80129 | AK223185 | Chr21 |

**Table 3 Genes highly down regulated in Down's Syndrome samples (T21)**

| **Marker** No | **SeqName** | **Gene description / name** | **Seqcode** | **Accession** No. | **Position** |
|---|---|---|---|---|---|
| 40 | IGHV1-46 | Immunoglobulin heavy chain V gene | A 32 P190951 | J00240 | Chrl4 |
| 41 | CHCHD2 | Coiled-coil-helix-coiled-coil-helix domain-containing protein 2 | A 24 P400376 | AC006970 | Chr7 |
| 42 | POLK | DNA polymerase kappa | A 23 P386450 | Q9UBT6 | Chr2 |
| 43 | IHPK2 | Inositol hexakisphosphate | A 23 P301133 | Q9UHH9-2 | Chr3 |
| 44 | ZNF625 | Zinc finger protein 625 | A 23 P4850 | BC101591 | Chrl9 |
| 45 | LEPR | leptin receptor | A 23 P161135 | | |
| 46 | BVES | Blood vessel epicardial substance | A 23 P502783 | AF124512 | Chr6 |
| 47 | C13orf30 | Uncharacterized protein C13orf30 | A 32 P332551 | AK098238 | Chrl3 |
| 48 | ING5 | Inhibitor of growth | A 23 P124202 | AK128322 | Chr2 |
| 49 | ZC3H5/UNK | Zinc finger CCCH domain-containing | A 32 P514790 | AB051540 | Chrl7 |
| 50 | RHO | Rhodopsin (Opsin-2). | A 23 P57950 | AB065668 | Chr3 |
| 51 | DMC1 | Meiotic recombination protein | A 23 P361381 | AL022320 | Chr22 |
| 52 | CD1B | CD lb molecule | A 23 P351844 | NM 001764.2 | Chr 1 |
| 53 | DKFZp761E19 8 | Putative uncharacterized | A 23 P24424 | AL834269 | Chrll |
| 54 | DNAH8 | Ciliary dynein heavy | A 23 P145159 | AF527621 | Chr 6 |

## Claims

1. A method of diagnosing Down's Syndrome the method comprising identifying an increased amount of at least one diagnostic marker in a sample of blood, serum or plasma from a patient compared to the normal expression of the marker, **characterised in that** the diagnostic marker is Trypsin-like serine protease LOC646960 protein and wherein the patient is a pregnant woman.

## Patentansprüche

1. Verfahren zur Diagnose von Down-Syndrom, wobei das Verfahren Identifizieren einer erhöhten Menge wenigstens eines diagnostischen Markers in einer Blut-, Serum- oder Plasmaprobe eines Patienten im Vergleich mit der normalen Expression des Markers umfasst, **dadurch gekennzeichnet, dass** der diagnostische Marker trypsinartiges Serinprotease LOC646960-Protein ist, und wobei der Patient eine Schwangere ist.

## Revendications

1. Procédé pour le diagnostic du Syndrome de Down, le procédé comprenant l'identification d'une quantité accrue d'au moins un marqueur de diagnostic dans un échantillon de sang, de sérum ou de plasma prélevé chez un patient, comparé à l'expression normale du marqueur, **caractérisé en ce que** le marqueur de diagnostic est une protéine appelée sérine protéase de type Trypsine LOC646960, et dans lequel le patient est une femme enceinte.
